(19) Europäisches Patentamt
European Patent Office
Office européen des brevets

(11) Numéro de publication : **0 162 771**
**B1**

(12) **FASCICULE DE BREVET EUROPÉEN**

(45) Date de publication du fascicule du brevet : **10.02.88**

(51) Int. Cl.[4] : **C 12 P 7/18** // (C12P7/18, C12R1:12)

(21) Numéro de dépôt : **85400887.7**

(22) Date de dépôt : **07.05.85**

(54) **Procédé de fabrication du 2,3 butane diol par fermentation aérobic d'un substrat par des souches de bacillus polymyxa.**

(30) Priorité : **18.05.84 FR 8407710**

(43) Date de publication de la demande : **27.11.85 Bulletin 85/48**

(45) Mention de la délivrance du brevet : **10.02.88 Bulletin 88/06**

(84) Etats contractants désignés : **AT BE CH DE GB IT LI LU NL SE**

(56) Documents cités :
**US-A- 1 899 156**
**US-A- 2 085 003**


(73) Titulaire : **SOCIETE CHIMIQUE DES CHARBONNAGES SA**
**Tour Aurore Cédex 5**
**F-92080 Paris la Défense 2 (FR)**

(72) Inventeur : **Wilhelm, Jean-Luc**
**15, rue Velane**
**F-31000 Toulouse (FR)**
Inventeur : **Fages, Jacques**
**Quartier de Saguens Route de Cugnaux**
**F-31120 Portet Sur Garonne (FR)**

(74) Mandataire : **Fourquet, Antoinette**
**CdF Chimie S.A. Service Propriété Industrielle B.P. No. 57**
**F-62670 Mazingarbe (FR)**


**EP 0 162 771 B1**


Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

**Description**

La présente invention concerne un procédé de fabrication de 2,3 butane diol par fermentation aérobie d'un substrat à l'aide de Bacillus Polymyxa.

Le 2,3 butane diol est un diol de formule $C_4O_2H_8$ qui existe sous trois formes isomères :

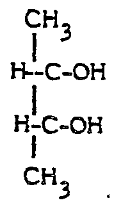

meso 2,3 butane diol

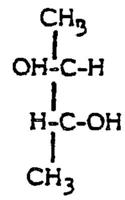

D(−) 2,3 buane diol isomère levogyre

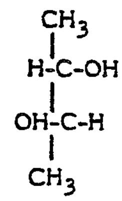

L(+) 2,3 butane diol isomère dextrogyre

Le 2,3 butane diol est essentiellement utilisé pour la préparation de 1,3 butadiène qui est une des matières premières pour la fabrication de caoutchoucs. Il peut également être une source de monomères tels que le méthylvinyl carbinol ou le méthylvinyl cétone. On peut l'utiliser à la place du glycérol dans la fabrication de résines polyester. Le 2,3 butane diol sous forme d'isomère levogyre peut être utilisé comme antigel. On peut également l'utiliser ainsi que l'isomère meso comme agent d'humidification dans les colles, la gélatine, la caséine, le tabac, le liège, les fibres textiles, etc.

Il est connu de préparer un 2,3 butane diol par fermentation à l'aide de Bacillus Polymyxa. Il est en particulier connu d'utiliser le Bacillus Polymyxa NRRL B 510 obtenu par Northern regional research laboratory. En effet, le Bacillus Polymyxa a l'avantage de donner du 2,3 butane diol sous forme levogyre pratiquement pure ce qui permet de l'utiliser comme antigel. On a fait des essais de fermentation aérobie sur de nombreux substrats : glucose, fructose, saccharose, amidon, mélasse de betterave, amidon de maïs, broyat de blé, liqueurs sulfitiques, petit lait, jus noir de sucrerie. Mais les rendements obtenus même avec le sucre comme substrat ne sont pas assez élevés pour que l'on puisse penser utiliser le procédé à l'échelle industrielle.

La présente invention concerne un procédé qui permet d'obtenir du 2,3 butane diol avec un bon rendement.

La présente invention concerne un procédé de fabrication de 2,3 butane diol par fermentation aérobie d'un substrat par des souches de Bacillus Polymixa, en particulier de Bacillus Polymyxa NRRL B 510, caractérisé par le fait que le substrat est constitué par du jus de topinambour n'ayant subi aucun traitement d'hydrolyse préalable.

Il était connu d'utiliser du jus de topinambour comme substrat pour la fermentation en particulier pour la fabrication d'éthanol. L'emploi de jus de topinambour est avantageux car c'est un aliment complet. En effet, il contient à la fois des sucres et des minéraux. Les sucres présents dans le jus de topinambour sont l'inuline à raison de 2/3 environ et le saccharose à raison de 1/3 environ. Il y a également du glucose et du fructose mais en faible quantité. Pour effectuer la fermentation du jus de topinambour à l'aide des microorganismes utilisés jusqu'à présent, il était nécessaire de l'hydrolyser pour dépolymériser l'inuline. Il était donc nécessaire d'effectuer une opération supplémentaire coûteuse.

Selon la présente invention, on a trouvé que le Bacillus Polymyxa permettait la fermentation du jus de topinambour sans qu'il soit nécessaire de l'hydrolyser. On obtient sans dépolymérisation préalable à la fermentation des rendements analogues à ceux obtenus avec d'autres substrats.

Selon l'invention, on ajoute de préférence au jus de topinambour un extrait de levure en quantité inférieure ou égale à 1 g/l. L'extrait de levure ajouté est une source de biotine, vitamine qui améliore le rendement de la fermentation. On a constaté que des quantités assez faibles avaient une action notable sur le rendement. Des quantités supérieures à 1 g/l n'apportent pas d'amélioration supplémentaire.

Pour effectuer la fermentation selon l'invention, on introduit dans un fermentateur du jus de topinambour. On utilise de préférence du jus brut de première pression qui est le plus simple à obtenir. Au préalable, le jus de topinambour a été stérilisé par passage à l'autoclave pendant environ 1 heure à une température de 105 °C ou pendant 15 minutes à une température de 120 °C. On ajoute éventuellement au jus de topinambour stérilisé l'extrait de levure en quantité inférieure ou égale à 1 g/l.

La quantité d'inoculum introduite est comprise entre 1/10 et 1/20 du volume du jus de topinambour. On fait passer ensuite de l'oxygène ou de l'air. Par exemple on peut introduire de l'air à raison de 0,5 volume d'air par volume de liquide et par minute (en abrégé V. V. M.). De préférence, pendant toute la durée de la fermentation, la température est réglée à une valeur comprise entre 25 et 32 °C et le pH maintenu entre 5,8 et 6,8 par addition d'ammoniaque. On maintient également le mélange sous agitation. La fermentation dure une cinquantaine d'heures. Lorsque la fermentation est terminée, on sépare le 2,3 butane diol du jus de fermentation et de la biomasse. Cette séparation peut être effectuée par préconcentration, entraînement à la vapeur et distillation.

On peut également effectuer une extraction (liquide-liquide) avec divers solvants (éther diéthylique,

n-butanol, acétate de butyle, butanediol diacétate, méthylvinylcarbinol). On peut transformer le 2,3 butanediol en 2,3 butane diol formal puis faire réagir ce dernier en milieu acide pour redonner le 2,3 butane diol.

Selon un premier mode de réalisation préféré du procédé de fermentation selon la présente invention, on effectue la fermentation aérobie en trois phases distinctes d'agitation, en maintenant une aération constante. Dans la première phase, on soumet le mélange à une forte agitation telle que le coefficient de transfert de l'oxygène à l'interface gaz-liquide soit compris entre 50 et 100 $h^{-1}$ dans la deuxième phase à une agitation telle que le coefficient de transfert soit compris entre 20 et 40 $h^{-1}$ et dans la troisième phase à une agitation plus réduite encore telle que le coefficient de transfert soit compris entre 10 et 20 $h^{-1}$.

En effet, dans la première phase, la présence d'oxygène dissous permet une croissance importante des bactéries. Cette croissance peut atteindre 10 bactéries/ml. Cette première phase permet donc d'acquérir une biomasse importante.

Dans la seconde phase, on réduit le transfert d'oxygène. En effet la voie métabolique conduisant au 2,3 butane diol est anaérobie et en réduisant la présence oxygène, on évite que les sucres du jus de topinambour ne soient utilisées uniquement pour faire des constituants cellulaires, du $CO_2$ et de l'eau par respiration et croissance du Bacillus Polymyxa.

Dans la troisième phase, on diminue encore l'agitation et par conséquent le transfert d'oxygène. On retarde ainsi la formation d'acétoïne à partir de 2,3 butane diol. A ce stade la concentration en éthanol n'augmente pas par suite de sa consommation et par conséquent, le rapport 2,3 butane diol/éthanol ne risque plus de diminuer.

Selon un deuxième mode de réalisation préféré de l'invention, on effectue la fermentation en deux phases d'aération distinctes, le débit d'air ou d'oxygène étant plus élevé dans la première phase que dans la seconde, et l'agitation étant maintenue constante dans les deux phases.

## Exemple 1

La teneur initiale en sucre de ce jus de topinambour est de 151 g/l.

On ajoute 1 g/l d'extrait de levure et 1/15 du volume du jus d'inoculum. Cet inoculum est obtenu par fermentation fortement aérée de 24 heures de jus de topinambour. On introduit de l'air dans le fermentateur à raison de 0,5 VVM. Dans une première phase d'une durée de 18h 40 minutes, on agite le mélange avec un agitateur tournant à une vitesse de 560 tours par minute (r. p. m.) ce qui donne un coefficient de transfert de 60 $h^{-1}$.

Le coefficient de transfert ($k_La$) est déterminé par désoxygénation du milieu réactionnel, puis enregistrement de la remontée de la concentration en oxygène dissous (concentration désignée par $C_L$) lorsque l'on rétablit l'aération. $C_L$ est mesurée par une sonde à oxygène dissous de type ampérométrique. Le coefficient de transfert k a est la pente de la droite

$$L_n\ [1 - (CL/C^*)] = f(t)$$

où t est le temps et C* la concentration saturante en oxygène dissous.

Dans une seconde phase d'une durée de 22 h, la vitesse d'agitation est réduite à 380 r. p. m., ce qui correspond à un coefficient de transfert de 20 $h^{-1}$.

Dans une troisième phase, l'agitation est encore réduire jusqu'à 315 r. p. m. ce qui correspond à un coefficient de transfert inférieur à 15 $h^{-1}$. Cette phase dure 11 heures. On obtient une solution contenant 44 g/l de 2,3 butane diol ce qui correspond à un rendement en butane diol par rapport au sucre consommé de 31 %.

La figure 1 annexée indique la courbe d'évolution des différents composés au cours de la fermentation.

## Exemple 2

L'exemple 1 est répété mais la phase de fermentation est réalisée en deux étapes :

— une première étape de 6 heures d'aération intense dans laquelle on introduit de l'air à raison de 2,1 V. V. M.

— une deuxième étape de 50 heures dans laquelle on introduit de l'air à raison de 0,3 V. V. M.

L'agitation est maintenue à 350 r. p. m. pendant les 2 phases d'oxygénation.

On obtient une solution contenant 43 g/l de 2,3 butane diol ce qui correspond à un rendement en butane diol par rapport au sucre consommé de 37,4 %.

La figure 2 annexée donne la courbe d'évolution des différents composés au cours de la fermentation.

**Revendications**

1. Procédé de fabrication de 2,3 butane diol par fermentation aérobie d'un substrat par des souches

de Bacillus Polymyxa, en particulier de Bacillus Polymyxa NRRL 510 caractérisé par le fait que le substrat est constitué par du jus de topinambour n'ayant subi aucun traitement d'hydrolyse préalable.

2. Procédé de fabrication de 2,3 butane diol selon la revendication 1 caractérisé par le fait que l'on ajoute au jus de topinambour un extrait de levure en quantité inférieure ou égale à 1 g/l.

3. Procédé de fabrication de 2,3 butane diol par fermentation selon la revendication 1, caractérisé par le fait qu'on introduit dans le jus de topinambour préalablement stérilisé un inoculum en quantité comprise entre 1/10 et 1/20 du volume du jus de topinambour, on fait ensuite passer de l'oxygène ou de l'air, on règle pendant toute la durée de la fermentation la température à une valeur comprise entre 25 et 32 °C et le pH à une valeur comprise entre 5,8 et 6,8 par addition d'ammoniaque et on maintient le mélange sous agitation, puis lorsque la fabrication est terminée on sépare le 2,3 butane diol du jus de fermentation et de la biomasse.

4. Procédé de fabrication de 2,3 butane diol par fermentation selon l'une quelconque des revendications 1 à 3 caractérisé par le fait que l'on effectue la fermentation en trois phases distinctes d'agitation en maintenant une aération constante, l'agitation étant dans la première phase telle que le coefficient de transfert d'oxygène à l'interface gaz-liquide soit compris entre 50 et 100 $h^{-1}$, dans la deuxième phase telle que le coefficient de transfert soit compris entre 20 et 40 $h^{-1}$ et dans la troisième phase telle que le coefficient de transfert soit compris entre 10 et 20 $h^{-1}$.

5. Procédé de fabrication de 2,3 butane diol par fermentation selon l'une quelconque des revendications 1 à 3 caractérisé par le fait que l'on effectue la fermentation en deux phases d'aération distinctes, le débit d'air ou d'oxygène étant plus élevé dans la première phase que dans la seconde et l'agitation étant maintenue constante dans les deux phases.

**Claims**

1. Process for making 2,3-butanediol by aerobic fermentation of a substrate by strains of Bacillus polymyxa, in particular of Bacillus polymyxa NRRL 510, characterized by the fact that the substrate is Jerusalem artichoke juice which has not been subjected to any prior hydrolysis treatment.

2. Process for making 2,3-butanediol according to claim 1 characterized by the fact that an extract of yeast in a quantity less than or equal to 1 g/l is added to the Jerusalem artichoke juice.

3. Process for making 2,3-butanediol by fermentation according to claim 1 characterized by the fact that an inoculum in a quantity comprising between 1/10 and 1/20 of the volume of the Jerusalem artichoke juice is introduced into the previously sterilized Jerusalem artichoke juice, then oxygen or air is passed through, during the entire fermentation period the temperature is controlled at a value between 25 and 32 °C and the pH at a value between 5.8 and 6.8 by the addition of ammonia and the mixture is kept agitated, then, when the production is ended, the 2,3-butanediol is separated from the fermentation juice and the biomass.

4. Process for making 2,3-butanediol by fermentation according to any one of the claims 1 to 3 characterized by the fact that the fermentation is effected with three distinct phases of agitation whilst maintaining constant aeration, agitation in the first phase being such that the coefficient of oxygen transfer at the gas-liquid interface is between 50 and 100 $h^{-1}$, in the second phase such that the coefficient of transfer is between 20 and 40 $h^{-1}$ and in the third phase such that the coefficient of transfer is between 10 and 20 $h^{-1}$.

5. Process for making 2,3-butanediol by fermentation according to any one of the claims 1 to 3 characterized by the fact that the fermentation is effected with two distinct phases of aeration, the supply of air or of oxygen being higher in the first phase than in the second and the agitation being kept constant during the two phases.

**Patentansprüche**

1. Verfahren zur Herstellung von 2,3-Butan-diol durch aerobe Gärung eines Substrates durch Stämme von Bacillus Polymyxa, insbesondere Bacillus Polymyxa NRRL 510, dadurch gekennzeichnet, daß das Substrat aus Brühe von Topinambur, welche keiner vorherigen Hydrolysebehandlung unterworfen worden war, besteht.

2. Verfahren zur Herstellung von Butan-diol nach Anspruch 1, dadurch gekennzeichnet, daß man zu der Topinamburbrühe einen Hefextrakt in einer Menge unter oder gleich 1 g/l hinzufügt.

3. Verfahren zur Herstellung von 2,3-Butan-diol durch Gärung nach Anspruch 1, dadurch gekennzeichnet, daß man in die vorher sterilisierte Topinamburbrühe eine Impfsubstanz in einer Menge zwischen 1/10 und 1/20 des Volumens der Topinamburbrühe einbringt, sodann Sauerstoff oder Luft durchleitet, während der gesamten Dauer der Gärung die Temperatur auf einen Wert zwischen 25 und 32 °C und den pH-Wert durch Zugabe von Ammoniak auf einen Wert zwischen 5,8 und 6,8 einreguliert und die Mischung unter Rühren hält, sodann, wenn die Herstellung beendet ist, das 2,3-Butan-diol aus der Topinamburbrühe und der Biomasse abtrennt.

4. Verfahren zur Herstellung von 2,3-Butan-diol durch Gärung nach einem der Ansprüche 1-3,

dadurch gekennzeichnet, daß man die Gärung in drei getrennten Rührphasen unter Aufrechterhaltung einer konstanten Belüftung durchführt, wobei das Rühren in der ersten Phase derart erfolgt, daß der Sauerstoff-Übergangskoeffizient an der Gas-Flüssig-Grenzfläche zwischen 50 und 100 $h^{-1}$ beträgt, in der zweiten Phase derart ist, daß der Sauerstoff-Übergangskoeffizient zwischen 20 und 40 $h^{-1}$ beträgt und in der dritten Phase derart ist, daß der Sauerstoff-Übergangskoeffizient zwischen 10 und 20 $h^{-1}$ beträgt.

5. Verfahren zur Herstellung von 2,3-Butan-diol durch Gärung nach einem der Ansprüche 1-3, dadurch gekennzeichnet, daß man die Gärung in zwei getrennte Belüftungsphasen durchführt, wobei der Durchsatz von Luft oder Sauerstoff in der ersten Phase höher ist als in der zweiten Phase und das Rühren in den beiden Phasen gleichmäßig gehalten wird.

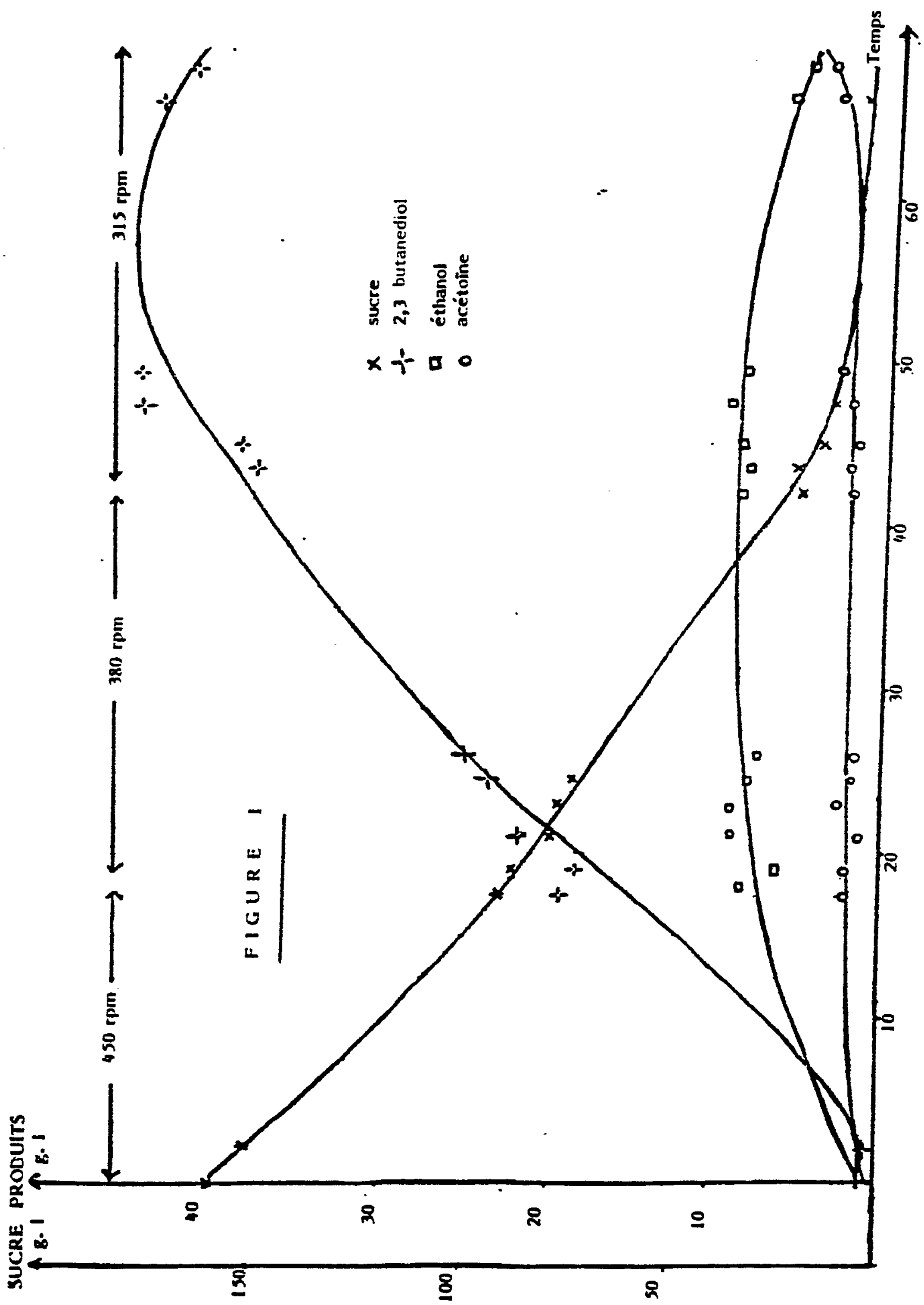
SUCRE g.l-1
PRODUITS g.l-1
150
100
50
40
30
20
10
450 rpm
380 rpm
315 rpm
x sucre
2,3 butanediol
□ éthanol
o acétoïne
10
20
30
40
50
60
Temps

FIGURE 1

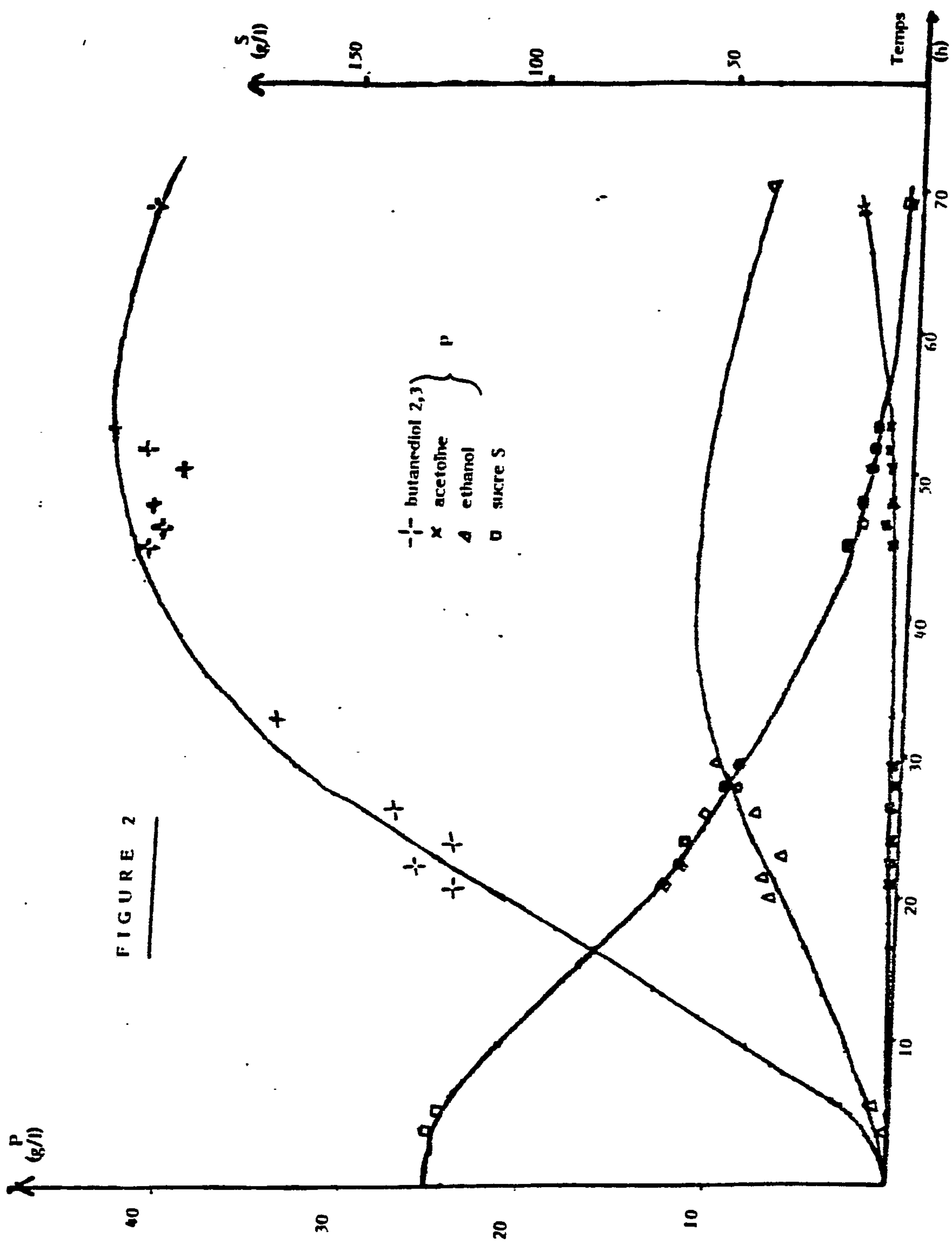
P (g/l)
40
30
20
10
S (g/l)
150
100
50
Temps (h)
10
20
30
40
50
60
70
butanediol 2,3
acetoine
ethanol
sucre S
P

FIGURE 2